# EUROPÄISCHE PATENTSCHRIFT

(11) **EP 2 637 967 B1**
(45) Veröffentlichungstag und Bekanntmachung des Hinweises auf die Patenterteilung: **21.10.2015**
(21) Anmeldenummer: 11778842.2
(22) Anmeldetag: 27.10.2011
(51) Int. Cl.: C01B 3/36, B01F 5/04, B01J 12/00, B01J 4/00, B01J 19/00, C07C 2/78, B01F 3/02, F23D 14/76, F23D 14/62, F23D 14/32, F23D 14/22

(54) **VERFAHREN UND VORRICHTUNG ZUR HERSTELLUNG VON ACETYLEN UND SYNTHESEGAS**
PROCESS AND APPARATUS FOR PREPARING ACETYLENE AND SYNTHESIS GAS
PROCÉDÉ ET DISPOSITIF POUR PRODUIRE DES ACÉTYLES ET UN GAZ DE SYNTHÈSE

(30) Priorität: 11.11.2010 US 412406 P
(43) Veröffentlichungstag der Anmeldung: 18.09.2013
(73) Patentinhaber: BASF SE, 67056 Ludwigshafen (DE)
(72) Erfinder: RUSS, Michael, 67354 Römerberg (DE); GROßSCHMIDT, Dirk, 68259 Mannheim (DE); RENZE, Peter, 68163 Mannheim (DE); VICARI, Maximilian, 67117 Limburgerhof (DE); NEUHAUSER, Horst, 67373 Dudenhofen (DE); EHRHARDT, Kai Rainer, 67346 Speyer (DE); WEICHERT, Christian, 67098 Bad Dürkheim (DE)
(86) Internationale Anmeldenummer: PCT/EP2011/068814
(87) Internationale Veröffentlichungsnummer: WO 2012/062584

(56) Entgegenhaltungen:
- WO-A1-2009/109473
- GB-A- 824 328
- GB-A- 951 100
- GB-A- 1 414 684
- GB-A- 1 482 975
- US-A- 3 551 512

## Beschreibung

Die vorliegende Erfindung betrifft ein verbessertes Verfahren zur Herstellung von Acetylen und Synthesegas durch partiellen Oxidation von Kohlenwasserstoffen in einem Reaktor, bei welchem man dem Reaktor einen den Kohlenwasserstoff enthaltenden Strom sowie einen den Sauerstoff enthaltenden Strom zuleitet sowie eine Vorrichtung zur Durchführung des erfindungsgemäßen Verfahrens.

Hochtemperaturreaktionen zur partiellen Oxidation von Kohlenwasserstoffen werden üblicherweise in einem Reaktorsystem aus Mischeinheit, Brenner und Quencheinrichtung durchgeführt.

Als Beispiel für eine solche Partialoxidation im Hochtemperaturbereich ist die Herstellung von Acetylen und Synthesegas durch partielle Oxidation von Kohlenwasserstoffen zu nennen. Diese wird beispielsweise in DE 875198, DE 1051845, DE 1057094 und DE 4422815 beschrieben.

Hierin werden die für das BASF-Sachsse-Bartholome-Acetylenverfahren üblicherweise eingesetzten Mischer/Brennerblock/Feuerraum/Quench-Kombinationen - im Folgenden, wenn auf die Kombination Bezug genommen wird, vereinfacht als "Reaktor" bezeichnet - erläutert.

Die Ausgangsstoffe wie beispielsweise Erdgas und Sauerstoff werden dabei getrennt aufgeheizt, üblicherweise bis hin zu 600°C. In einer Mischzone werden die Reaktanden intensiv vermischt und nach Durchströmen eines Brennerblocks zur exothermen Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch ihre zylinderförmige Geometrie des Feuerraums aus. Der Brennerblock weist vorzugsweise hexagonal angeordnete Durchführungsbohrungen auf. In einer Ausführungsform sind z.B. 127 Bohrungen ä 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in der die Flamme der acetylenbildenden partiellen Oxidationsreaktion stabilisiert wird, ist ebenfalls von zylindrischem Querschnitt, ist wassergekühlt und entspricht im Erscheinungsbild dem eines kurzen Rohres (von z.B. 180 bis 533 mm Durchmesser und 380 bis 450 mm Länge). In Höhe derfeuerraumseitigen Oberfläche des Brennerblocks wird sogenannter Hilfssauerstoff dem Reaktionsraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind außerhalb von dessen Umfang Quenchdüsen auf einem oder mehreren Quenchverteilerringen installiert, die das Quenchmedium, z.B. Wasser oder Öl, mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe, die reagierende Strömung extrem schnell auf ca. 100 °C (Wasserquench) und 200 °C (Ölquench) abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Die im aktuellen Produktionsmaßstab eingesetzten Acetylenbrenner zeichnen sich durch eine zylinderförmige Geometrie des Feuerraums aus. Die Einsatzstoffe werden über einen Diffusor vorgemischt und unter Vermeidung von Rückvermischung dem Brennerblock über hexagonal angeordnete Durchführungsbohrungen zugeführt. Bei den bekannten Verfahren erfolgt die Vormischung der Einsatzstoffe im Mischdiffusor in einem relativ großen Volumen und unter hohen Vorwärmtemperaturen.

Bei dem beschriebenen technischen Verfahren entsteht neben Acetylen im Wesentlichen Wasserstoff, Kohlenmonoxid und Ruß. Die in der Flammenfront gebildeten Rußpartikel können als Keime an der feuerraumseitigen Oberfläche des Brennerblocks anhaften, worauf es zum Aufwachsen, Ablagern und Anbacken von Koksschichten kommt, wodurch das Verfahren in seiner Effektivität nachhaltig beeinträchtigt wird.

In den bestehenden Produktionsverfahren mit Öl- und Wasserquench werden diese Ablagerungen periodisch im Bereich der feuerraumseitigen Oberfläche des Brennerblocks mittels einer Stocherreinrichtung mechanisch abgereinigt. Hierfür ist eine aufwändige Steuerung der Stochereinrichtung nötig (Ullmann's Encyclopedia of Industrial Chemistry, 5th Edition, Volume A1, pages 97 - 144) und zudem wird die jeweilige Einsatzzeit des Mechanismus durch die thermische Belastung im Brennraum limitiert

Es hat nicht an Versuchen gefehlt, den Nachteil des Anbackens von Koksschichten an der feuerraumseitigen Oberfläche des Brennerblocks zu vermeiden. So offenbart die Lehre der DE 2307300 das Injizieren einer gasförmigen Substanz in den Reaktor in einer Region zwischen maximaler Temperatur und Ort des Abschreckens (Anspruch 1). Hierbei soll es zu Reaktionen zwischen den zugegebenen Gasen und freien Radikalen kommen, wodurch die Koksbildung reduziert werden soll (Beschreibung, Seite 8, zweiter Absatz).

In der DE 3904330 A1 wird ein Verfahren zur Herstellung von Acetylenruß durch thermische Zersetzung von Acetylen beschrieben. Es wird hier bei diesem Verfahren, welches sich von dem Verfahren zur Herstellung von Acetylen deutlich unterscheidet (z.B. keine partielle Oxidation), erwähnt, dass man ggf. einen Inertgasstrom einleitet.

Die DE 1148229 beschreibt ein Verfahren zum Betreiben von Spaltkammern zur Behandlung von Kohlenwasserstoffen, wobei eine Spülung mit Wasserdampf vorgesehen ist und eine Kühlung der Wand zu einem Wasserschleier führen soll (Anspruch 1). Genauere Angaben über die Art der Ausführung der Spülung werden nicht gemacht. Es handelt sich bei dem vorgestellten Verfahren nicht um eine partielle Oxidation (POx), als Spülmedium wird flüssiges Wasser eingebracht und ein zusätzliches Beimischen eines Oxidationsmittels (z.B. Sauerstoff) zum Spülmedium ist nicht vorgesehen. Desweiteren wird im axialen Verlauf der Spaltkammer nur an maximal einer Stelle ein Spülmedium injiziert.

In der DE 2007997 wird beschrieben, wie ein Ölfilm an der Innenwand der Reaktionskammer eine Verkokung vermeiden soll (Seite 2, erster Absatz). Ein Ölfilm in einem Feuerraum neigt per se jedoch zur Verkokung. Daher kann ein kohlenwasserstoffhaltiges (Mineral-)Öl als Spülmedium bei der vorliegenden Herausforderung ausgeschlossen werden.

In der GB 951100 A wird ein Apparat für die Durchführung einer Partialoxidation beschrieben, bei welchem durch Leitungen ein oxidierendes Gas zur Flammenstabilisierung zugeführt wird.

Die WO 2009/109473 beschreibt ein Verfahren, bei welchem die Einsatzstoffströme getrennt und nicht vorvermischt in den Reaktionsraum eingeleitet werden.

Die in den genannten Schriften offenbarten Verfahren zur Verhinderung bzw. Verringerung von unerwünschter Koksbildung befriedigen jedoch nicht bezüglich einer effektiven Anwendung beim Verfahren zur Herstellung von Acetylen. So beziehen sich einige der Schriften wie erläutert auf andere Reaktionen, wo gänzlich andere Bedingungen vorliegen und eine Übertragbarkeit nicht gegeben ist. So ist die bei dem erfindungsgemäßen Verfahren vorliegende partielle Oxidation in ihrer Charakteristik sehr anspruchsvoll: die Verweilzeiten spielen eine besonders große Rolle, der Abbruch der Reaktion muss sehr exakt erfolgen und die Zugabe von Fremdstoffen wie auch beispielsweise eines Spülgases oder Oxidators kann die Reaktion hinsichtlich ihres Ortes sowie ihrer Geschwindigkeit sehr schnell verschieben und damit zu einem Ausbeuteverlust führen.

Weiterhin finden sich im Stand der Technik nur allgemeine, eher aufgabenhafte Aussagen über die technische Ausgestaltung. Ferner finden sich im Stand der Technik Erläuterungen mit Bezug auf eine Verkokung an den Seitenflächen, welche den Feuerraum begrenzen und nicht auf die feuerraumseitige Oberfläche des Brennerblocks, welche üblicherweise senkrecht zu diesen Seitenflächen steht.

Weiterhin werden keine strömungsmechanischen und reaktionstechnischen Geometrievorschriften vorgestellt anhand derer eine effektive Minimierung des Spülmediums und damit eine Minimierung von Betriebskosten und eine Optimierung der Produktausbeute erfolgen könnte.

Es stellte sich somit die Aufgabe, ein verbessertes Verfahren zur partiellen Oxidation von Kohlenwasserstoffen zu finden, welches in verfahrenstechnisch einfacher Art und Weise Anbackungen und Ablagerungen an der feuerraumseitigen Oberfläche des Brennerblocks unterbindet, damit sich ein mechanisches Säubern dieser feuerraumseitigen Oberfläche des Brennerblocks erübrigt und somit eine periodische Abreinigung mittels thermisch hochbelasteter und aufwändig zu steuernden mechanischen Stochereinrichtungen entfällt.

Demgemäß wurde ein Verfahren zur Herstellung von Acetylen und Synthesegas durch partielle Oxidation von Kohlenwasserstoffen mit Sauerstoff gefunden, wobei die Ausgangsgase umfassend einen kohlenwasserstoffhaltigen Strom und einen sauerstoffhaltigen Strom zunächst getrennt vorerhitzt und anschließend in einer Mischzone vermischt werden und nach Durchströmen des Brennerblocks in dem Feuerraum zur Reaktion gebracht und anschließend schnell abgekühlt werden, welches dadurch gekennzeichnet ist, dass man die effektive feuerraumseitige Oberfläche des Brennerblocks, welche sich aus der ursprünglichen feuerraumseitigen Oberfläche des Brennerblocks, wobei die durch die Bohrungen für das Einsatzstoffgemisch entfallenden Teile dieser Fläche in Abzug gebracht werden, ergibt, mit einem Spülgasstrom belegt, man diesen Spülgasstrom mittels mehrerer Bohrungen durch den Brennerblock einbringt, wobei das gemittelte Verhältnis von effektiver Oberfläche des Brennerblocks und Anzahl dieser Bohrungen im Brennerblock für den Spülgasstrom in einem Bereich von 5 bis 100 cm² liegt, wobei das gemittelte Verhältnis von effektiver Oberfläche des Brennerblocks und Anzahl dieser Bohrungen im Brennerblock für den Spülgasstrom aus dem Verhältnis aus der gesamten effektiven Oberfläche des Brennerblocks zur gesamten Anzahl der Bohrungen für das Spülmedium gebildet wird, wobei man den durch die Bohrungen geführten Spülgasstrom mittels Verteilervorrichtungen derart umlenkt, dass 70 bis 100 Vol% des zugeführten Spülgasstromes parallel zur feuerraumseitigen Oberfläche des Brennerblocks geführt werden.

Das erfindungsgemäße Verfahren lässt sich auf allgemein bekannte Verfahren zur Herstellung von Acetylen und/oder Synthesegas durch partielle Oxidation anwenden. Als Einsatzstoffe eigenen sich bei den Kohlenwasserstoffen bevorzugt u.a. Alkane, Alkene, Erdgase, Leichtbenzin und Gemischen dieser mit beispielsweise CO₂, Synthesegas. Der sauerstoffhaltige Strom kann beispielsweise über Sauerstoff aber auch Gemische enthaltend Sauerstoff sowie beispielsweise CO₂, H₂O, N₂, und/oder Edelgasen, zugeführt werden.

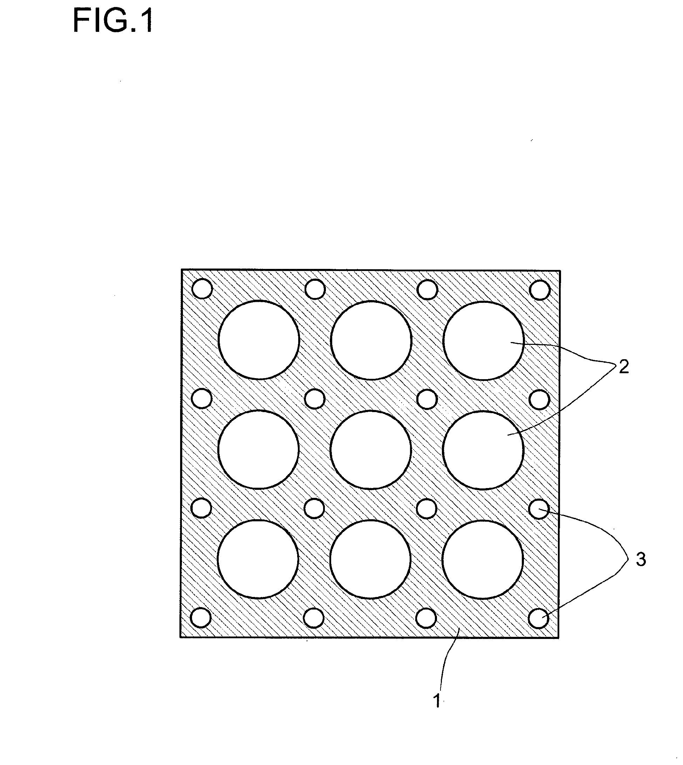

In Figur 1 sieht man einen Ausschnitt aus dem erfindungsgemäßen Brenner. Man erkennt hier die feuerraumseitige Oberfläche des Brennerblocks (1) mit zwei Arten von Bohrungen, welche eine Zufuhr von Stoffströmen in den Feuerraum ermöglichen. Es sind Bohrungen (2) für die Zufuhr des Einsatzstoffgemisches sowie Bohrungen (3) für die Zufuhr des Spülgases dargestellt, wobei die Bohrungen für das Spülgas in ihrem freien Querschnitt kleiner gehalten sind als die Bohrungen für das Einsatzstoffgemisch. Bevorzugt handelt es sich bei den Bohrungen um kreisförmige Querschnitte, es können jedoch auch andere Geometrien zur Anwendung kommen. Wie der Figur 1 zu entnehmen ist, übersteigt bevorzugt die Anzahl der Bohrungen für das Spülgas in Bezug auf ein betrachtetes Flächensegment die Anzahl der Bohrungen für das Einsatzstoffgemisch.

Anhand von Figur 1 kann auch der Begriff von "effektiver Oberfläche des Brennerblocks" erläutert werden. Diese ist hier schraffiert dargestellt. Sie ergibt sich aus der ursprünglichen feuerraumseitigen Oberfläche des Brennerblocks, wobei die durch die Bohrungen für das Einsatzstoffgemisch entfallenen Teile dieser Fläche in Abzug gebracht werden.

Erfindungsgemäß kann die Anzahl der Bohrungen (3) in Bezug auf einen Teil der feuerraumseitigen Oberfläche des Brennerblocks variieren. So kann es sich beispielsweise besonders empfehlen, in Nähe der Außenwand (d.h. die den Feuerraum begrenzenden Wand, welche in der Regel senkrecht zur Oberfläche des Brennerblocks steht) eine höhere Dichte solcher Bohrungen (3) für das Spülgas vorzusehen, um in Nähe dieses relativ kalten Bereiches, wo eine erhöhte Gefahr der Koksbildung besteht, durch verstärkte Zufuhr von Spülgas dem unerwünschten Effekt der Verkokung zu begegnen. Hierbei kann der Volumenstrom des zugeführten Spülgases im Randbereich bevorzugt das 1,2 bis 10 fache des mittleren Volumenstroms an Spülgas (durch Mittelwertbetrachtung sämtlicher Spülgaszuführungen durch alle Bohrungen 3 leicht bestimmbar) betragen. Unter Randbereich versteht man hier bei die äußeren ca. 10 % - 20 % der radialen Feuerraumabmessung (bei kreisförmigen Querschnitten entspricht diese Abmessung dem Durchmesser). Um das "gemittelte Verhältnis von effektiver Oberfläche des Brennerblocks und Anzahl dieser Bohrungen im Brennerblock für den Spülgasstrom" zu ermitteln, stellt man eine Gesamtbetrachtung an. Es wird hierbei das Verhältnis aus der gesamten (feuerraumseitigen) effektiven Oberfläche des Brennerblocks zur gesamten Anzahl der Bohrungen für das Spülmedium gebildet. Dieses gemittelte Verhältnis liegt bei 5 bis 100 cm², bevorzugt 5 bis 50 cm². Wie bereits vorstehend geschildert, kann dieses Verhältnis über die Oberfläche des Brennerblocks variieren.

Die feuerraumseitige Oberfläche des Brennerblocks, welche erfindungsgemäß gespült wird, umfasst somit die (feuerraumseitige) effektive Oberfläche des Brennerblocks abzüglich der durch die Bohrungen für das Spülgas verloren gegangenen Flächenanteile. Erfindungsgemäß wird eine Verkokung und Aufwachsung von Russ auf dieser Oberfläche vermieden. Erfindungsgemäß wird der hierzu eingesetzte Spülgasstrom über eine oder mehrere Zuleitungen den entsprechenden Bohrungen im Brennerblock zugeführt und tritt an der feuerraumseitigen Oberfläche der Brennerblocks aus.

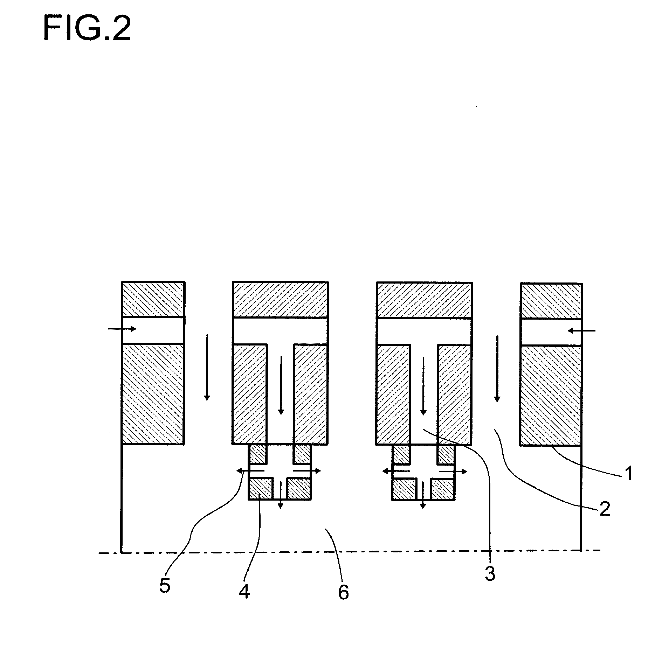

Es ist ein wesentliches Ziel des erfindungsgemäßen Verfahrens, die Menge des einzubringenden Spülstromes möglichst gering zu halten, um eine signifikante Abweichung der Stöchiometrie der Einsatzstoffe zu verhindern, wodurch die Acetylenausbeute verringert werden würde. Zudem ist die Menge des eingesetzten Spülmediums aus Gründen der Wirtschaftlichkeit zu minimieren. Hierbei soll gleichzeitig eine hohe Effektivität bei der Verhinderung der Verkokung der feuerraumseitigen Oberfläche des Brennerblocks erhalten bleiben. Um diesen Effekt zu erzielen ist ein möglichst gleichmäßiger Spülfilm auf der feuerraumseitigen Oberfläche des Brennerblocks zu gewährleisten. Ein Beispiel für eine konstruktive Lösung findet sich in Figur 2. Hier sieht man wiederum einen Ausschnitt aus einer erfindungsgemäßen Vorrichtung. Man erkennt die feuerraumseitige Oberfläche des Brennerblocks (1) mit Bohrungen für die Zufuhr des Einsatzstoffgemisches (2) sowie Bohrungen für die Zufuhr des Spülgases (3). An diese Bohrungen (3) grenzt an ihrem Ende, also zum Feuerraum (6) gerichtet, eine Verteilervorrichtung (4) für das Spülgas. Die Pfeile (5) deuten Richtungen an, in welcher sich das Spülgas verteilt.

In einer bevorzugten Ausführungsform werden große Anteile des zugegebenen Spülgases parallel zur feuerraumseitigen Oberfläche des Brennerblocks geführt. Es empfiehlt sich hier ein Anteil von 20 bis 100 Vol%, besonders 70 bis 100 Vol%. Hierbei kann es sich besonders empfehlen, dass der der aus der Verteilervorrichtung austretende Spülgasstrom in Bezug auf die Mittelpunktsachse der Zuleitung durch eine Vielzahl über den äußeren Umfang des Verteilers bevorzugt gleichmäßig angeordnete Öffnungen radial verteilt wird, wodurch eine gleichmäßige (bevorzugt parallele) Verteilung des Spülgases über die feuerraumseitige Oberfläche des Brennerblocks gewährleistet wird. Hierbei weist eine Verteilervorrichtung bevorzugt mindestens drei Öffnungen für den Austritt des Spülgases auf, üblicherweise befinden sich hier jedoch deutlich mehr Öffnungen, um eine gute Vergleichmäßigung der (radialen) Verteilung zu erzielen. Durch diese spezielle Anordnung legt sich der Spülfilm direkt und gleichmäßig geschlossen auf die zu spülende feuerraumseitige Oberfläche der Brennerblocks, was eine Durchdringung von Rußpartikeln auf diese Wand am effektivsten verhindert. Zudem werden durch ein paralleles Auströmen des Spülfilms zur zu spülenden feuerraumseitige Oberfläche der Brennerblocks anströmende Rußpartikel in Richtung der Bohrungen für die Zufuhr des Einsatzstoffgemisches transportiert und in der dort austretenden Flamme umgesetzt, was ebenfalls eine Verhinderung von Anbackungen auf der feuerraumseitige Oberfläche der Brennerblocks erfindungsgemäß begünstigt.

Bei der Zugabe des Spülgases werden bevorzugt 0,05 bis 1 Nm³ pro Stunde und Bohrung zugegeben, besonders bevorzugt 0,1 bis 0,6 Nm³ pro Stunde und Bohrung. Diese Wertebereiche ergeben sich zum einen aus der erfindungsgemäßen Vorgabe hoher Austrittsgeschwindigkeiten und damit hoher Impulse des Spülmediums (50 - 200 m/s), zum anderen aus der fertigungstechnischen Vorgabe begrenzt kleiner Austrittsöffnungen (Durchmesser ca. 0,1 - 1 mm), der wirtschaftlichen Anforderungen möglichst geringer Mengen an Spülmedium und der verfahrensbedingter Vorgabe möglichst die Stöchiometrie der Einsatzstoffe nur geringförmig zu verändern. Unter diesen Vorbedingungen hat sich eine Dichte an Spülbohrungen von 5 bis 100 cm ² pro Bohrung als sinnvoll ergeben.

In einer bevorzugten Ausführungsform kann das Spülgas über die gesamte effektive Oberfläche des Brennerblocks betrachtet in unterschiedlicher Menge zugeleitet werden, wobei in Nähe der den Feuerraum begrenzenden Wand bevorzugt eine höhere Menge zugeführt wird. Hierdurch kann die Rußabscheidung weiter verringert werden, da in eher kühleren Bereichen wie in Nähe der erwähnten begrenzenden Wand die Tendenz zur Verkokung höher ist.

Für die Ausgestaltung der Verteilervorrichtung (4) kommen unterschiedliche Ausgestaltungen in Betracht. So können beispielsweise kugelförmige, pyramidenförmige, kegelförmige oder würfelförmige Geometrien angewendet werden. Besonders können sich auch beispielsweise pyramidenförmige Geometrien empfehlen, wobei die Spitze in Richtung des Feuerraumes orientiert ist. Durch entsprechenden Anordnung von kleinen Öffnungen in der Verteilervorrichtung kann das Spülgas entsprechend aufgegeben werden. Dem bevorzugten Erfordernis der vorwiegend parallelen Strömungsführung in Bezug auf die Oberfläche des Brennerblocks kann beispielsweise durch eine entsprechende Anordnung dieser kleinen Öffnungen in der Verteilervorrichtung Rechnung getragen werden. In der Regel sollte man diese kleinen Öffnungen in ihren Maßen möglichst klein halten, wobei auch der gegebene Vordruck des Spülgases zu beachten ist.

Erfindungsgemäß befinden sich eine Vielzahl oder sämtliche Austrittsöffnungen aus den Verteilerkörpern (4) in unmittelbarer Nähe zu der zu spülenden Oberfläche des Brennerblocks (so beispielsweise in Figur 2 zu sehen).

Bei dem erfindungsgemäßen Verfahren kann das gewünschte Spülprinzip je nach eingesetztem Spülmedium auf zwei unterschiedlichen Mechanismen beruhen, welche auch beide gemeinsam zur Wirkung kommen können, wodurch sich besondere Synergismen ergeben.

Zum einen ein reines, wie zuvor beschriebenes, impulshaftes Spülen der Strömungsgrenzschicht. Dies führt zur Vermeidung sowohl des Eindringens von festen Koks-/Rußpartikeln als auch des Anhaftens und Aufwachsens von Ruß/Koksschichten. Die Geschwindigkeit des Spülfilms über der Spülfläche (feuerraumseitige Oberfläche des Brennerblocks) soll bevorzugt möglichst groß sein, um aus der Rektionszone zirkulierende Kokspartikel am Anhaften auf der Oberfläche zu hindern.

Zum Zweiten besteht die Möglichkeit durch das Spülmedium in Nähe der feuerraumseitigen Oberfläche des Brennerblocks eine oxidierende Atmosphäre zu schaffen, welche ein Aufwachsen/Anhaften von Ruß/Koks dadurch vermeidet, dass mögliche Ablagerungen mit Hilfe des im Spülmedium enthaltenen Oxidators umgesetzt/verbrannt/oxidiert werden, bzw. deren Bildung soweit verlangsamt, dass eine Bildung der festen Phase aus kinetischen Gründen verhindert wird.

Erfindungsgemäß kann zur Erzeugung des Spülimpulses jedwelches Spülmedium in Betracht kommen, bevorzugt Sauerstoff. Die Temperaturen des Spülmediums liegen bevorzugt unter 500°C, besonders bevorzugt in einem Bereich von 100 bis 300°C. Hierbei beziehen sich die genannten Temperaturen auf die Eintrittstemperatur des Spülmediums in den Feuerraum. Bei der zuvor beschriebenen oxidativen Spülwirkung kommen bevorzugt als Spülgas bei gegebenen verfahrenstechnischen Randbedingungen potentiell oxidierende Medien bevorzugt Wasserdampf, Sauerstoff, CO2, CO und Gemische daraus sowie aus weiteren Inertanteilen in Betracht. Auch die Verwendung von Luft als Spülmedium kann sich empfehlen.

In einer weiteren bevorzugten Ausgestaltung des erfindungsgemäßen Verfahrens belegt man zusätzlich noch die Innenwand des Feuerraums mit einem Spülgasstrom. Hierdurch kann das Verfahren insgesamt in seiner Effektivität noch gesteigert werden, da somit auch einer Verkokung dieser Wandung wirkungsvoll begegnet werden kann. Als Spülmedium eignen sich dabei im wesentlichen ebenfalls die vorstehend genannten Einsatzstoffe.

Weiterer Gegenstand dieser Erfindung ist eine für die Durchführung des erfindungsgemäßen Verfahrens geeignete Vorrichtung. Diese umfasst einen Reaktor enthaltend einen Brennerblock mit Feuerraum zur Acetylenherstellung, wobei der Brennerblock Bohrungen für die Zuleitung eines Spülgasstromes zur Spülung der feuerraumseitigen Oberfläche des Brennerblocks aufweist, wobei das gemittelte Verhältnis von effektiver Oberfläche des Brennerblocks und Anzahl dieser Bohrungen im Brennerblock für den Spülgasstrom in einem Bereich von 5 bis 100 cm² liegt.

Das erfindungsgemäße Verfahren bietet die Möglichkeit, Verkrustungen an der feuerraumseitigen Oberfläche des Brennerblocks zu vermeiden, wodurch mechanische Abreinigungstätigkeiten wie beispielsweise der Einsatz einer Stochereinrichtung vermieden werden können. Somit ist es dauerhaft gewährleistet, dass keine störenden Feststoffpartikel von relevanter Größe in den Gasstrom gelangen. Hierdurch ist es in einer bevorzugten Ausgestaltung des erfindungsgemäßen Verfahrens somit möglich, einen großen Teil der in dem Gas enthaltenen Wärme nach der Reaktion mittels eines nachgeschalteten Wärmetauschers zurückzugewinnen. Hierbei wird diese gewonnene Energie bevorzugt zur Dampferzeugung verwendet. Bevorzugt kann so aus dem hergestellten Produktgas ein Großsteil der Wärme zur Dampferzeugung rückgewonnen werden. Als Wärmetauscher eigenen sich beispielsweise Rohrbündelwärmetauscher. Bevorzugt wird hier der Gasstrom nach der Reaktion im Gegensatz zum herkömmlichen Verfahren nicht auf 100 °C bei Wasserquench bzw. 200 °C bei Ölquench, sondern schnell auf 600 - 1000 °C abgekühlt (gequencht). Dies führt noch zu keinen nennenswerten Ausbeuteverlusten an Acetylen durch Folgereaktionen. Anschließend wird dieses heiße Produktgas einem Wärmetauscher zugeführt, in welchem das Gas weiter abgekühlt wird. Die dabei frei werdende Energie wird zur Wärmerückgewinnung verwendet. Bevorzugt handelt es sich bei dem Wärmetauscher um einen Dampferzeuger, in dem wird indirekt das Produktgas weiter abgekühlt und sekundärseitig Dampf erzeugt, welcher weiter kommerziell oder prozesstechnisch nutzbar ist. Hierdurch wird die Effektivität des Verfahrens deutlich gesteigert. Es ist ein wesentlicher Vorteil bei der Ausgestaltung des erfindungsgemäßen Verfahrens, dass der Wärmetauscher unmittelbar nachgeschaltet werden kann, da hierbei keine Feststoffablagerungen zu Problemen führen. Neben einer Verwendung der bei der Abkühlung des Produktgases freiwerdenden Energie zur Dampferzeugung sind auch andere Möglichkeiten gegeben, diese Energie im Rahmen des Verfahrens zur Herstellung von Acetylen und Synthesegas zu nutzen.

Eine solche Verfahrensführung ist bei den bisherigen Verfahren so nicht wirtschaftlich realisierbar, da sich beim bisherigen mechanischen Abreinigungsverfahren Ruß- und Koksplatten im Zuge der Abreinigung von der feuerraumseitigen Oberfläche des Brennerblocks lösen, in Richtung Wärmetauscher transportiert werden und diesen mechanisch, abrasiv schädigen oder ganz oder teilweise verstopfen. Demgegenüber lässt sich erfindungsgemäß eine Ablagerung von Koks und Ruß auf der feuerraumseitigen Oberfläche des Brennerblocks vollständig unterbinden.

Das erfindungsgemäße Verfahren bietet eine verfahrenstechnisch einfache und effektive Möglichkeit zur wirkungsvollen Verringerung oder gar Verhinderung von Verkrustungen und Anbackungen von Koks an der feuerraumseitigen Oberfläche des Brennerblocks bei der Herstellung von Acetylen und / oder Synthesegas durch partielle Oxidation. Durch die vorstehend erläuterten, erfindungsgemäßen Merkmale kann hierbei bereits ein relativ geringer Spülgasstrom die Verkrustung wirkungsvoll verhindern, ohne dass die Effektivität der Reaktion nachhaltig beeinträchtigt wird. Hierbei kann in einer bevorzugten Ausführungsform die Zufuhr des Spülgases vorteilhafterweise in einzelnen Bereichen der feuerraumseitigen Oberfläche des Brennerblocks in unterschiedlicher Menge zugegeben werden, wodurch die Effektivität weiter gesteigert wird. Durch entsprechende Auswahl des Spülmediums kann neben einem "reinen Spülvorgang" basierend auf Impuls des Spülstroms ggf. auch bevorzugt eine oxidierende Atmosphäre geschaffen werden, wodurch die Ablagerungen weiter verringert werden können.

### Beispiele

Als Beispiel soll hier der Vergleich zwischen dem Betrieb eines Brennerblock / Feuerraums / Quenchs zur Acetylensynthese ("Standardreaktor") nach Stand der Technik und dem Betrieb eines Reaktors mit Brennerblock im Sinne der Erfindung betrachtet werden.

### Beispiel 1

Der Standardreaktor wurde zu Testzwecken mit herkömmlicher axialer und radialer Flammenstabilisierung betrieben. Der Feuerraum ist wassergekühlt ausgeführt.

Der Reaktordurchmesser beläuft sich bei den verwendeten Brennern auf 180 mm.

Der Brennerblock ist ausgeführt mit 19 hexagonal angeordneten Bohrungen a 27mm Innendurchmesser.

Der Standardreaktor und alle im Folgenden genannten Reaktoren wurden unter diesen Reaktionsbedingungen betrieben:
- Erdgasvolumenstrom: 600 Nm³/h
- Verhältnis Sauerstoffstrom zu Erdgasstrom = 0.62
- Vorwärmtemperatur der Gase: 550°C
- Acetylen im Volumen: 6.4%vol

Unter den gegebenen Versuchsbedingungen musste der Standardreaktor nach dem Auftreten von Flammenstabilitätsproblemen nach ca. 20 Betriebsstunden abgeschaltet werden. Bei Inspektion der feuerraumseitigen Oberfläche des Brennerblocks ist beim Standardreaktor eine ausgebildete, mehrere Zentimeter dicke Koksschicht auf der feuerraumseitigen Oberfläche des Brennerblocks auffindbar. Die Aufwachsrate der Koksschicht auf der feuerraumseitigen Oberfläche des Brennerblocks kann zu 2 mm/h bestimmt werden.

### Beispiel 2

Zum Vergleich wurde ein erfindungsgemäßer Reaktor mit Brennerblock eingesetzt, dadurch gekennzeichnet, dass die mit Brennerblocks mit einem Spülgasstrom belegt wurde, wobei das gemittelte Verhältnis von effektiver Oberfläche des Brennerblocks und Anzahl der Bohrungen im Brennerblock für den Spülgasstrom 7 cm² betrug. Dabei wurde 90 % des Spülgasstromes mittels Verteilervorrichtungen parallel zur feuerraumseitigen Oberfläche des Brennerblocks und radial im Bezug auf die Mittelpunktachse der Bohrungen im Brennerblock für die Zuleitung des Spülgasstroms zugeführt. Der Spülgasstrom bestand erfindungsgemäß aus Sauerstoff.

Beim Betrieb des erfindungsgemäßen Reaktors mit Brennerblock kommt es auf der feuerraumseitigen Oberfläche des Brennerblocks während einer Betriebszeit von 20h zu keinen Ablagerungen. Die Aufwachsrate der Koksschicht auf der feuerraumseitigen Oberfläche des Brennerblocks beträgt demnach 0 mm/h.

## Patentansprüche

1. Verfahren zur Herstellung von Acetylen und Synthesegas durch partielle Oxidation von Kohlenwasserstoffen mit Sauerstoff, wobei die Ausgangsgase umfassend einen kohlenwasserstoffhaltigen Strom und einen sauerstoffhaltigen Strom zunächst getrennt vorerhitzt und anschließend in einer Mischzone vermischt werden und nach Durchströmen des Brennerblocks in dem Feuerraum zur Reaktion gebracht und anschließend schnell abgekühlt werden, **dadurch gekennzeichnet, dass** man die effektive feuerraumseitige Oberfläche des Brennerblocks, welche sich aus der ursprünglichen feuerraumseitigen Oberfläche des Brennerblocks, wobei die durch die Bohrungen für das Einsatzstoffgemisch entfallenden Teile dieser Fläche in Abzug gebracht werden, ergibt mit einem Spülgasstrom belegt, man diesen Spülgasstrom mittels mehrerer Bohrungen durch den Brennerblock einbringt, wobei das gemittelte Verhältnis von effektiver Oberfläche des Brennerblocks und Anzahl dieser Bohrungen im Brennerblock für den Spülgasstrom in einem Bereich von 5 bis 100 cm² liegt, wobei das gemittelte Verhältnis von effektiver Oberfläche des Brennerblocks und Anzahl dieser Bohrungen im Brennerblock für den Spülgasstrom aus dem Verhältnis aus der gesamten effektiven Oberfläche des Brennerblocks zur gesamten Anzahl der Bohrungen für das Spülmedium gebildet wird, wobei man den durch die Bohrungen geführten Spülgasstrom mittels Verteilervorrichtungen derart umlenkt, dass 70 bis 100 Vol% des zugeführten Spülgasstromes parallel zur feuerraumseitigen Oberfläche des Brennerblocks geführt werden.

2. Verfahren nach Anspruch 1, **dadurch gekennzeichnet, dass** das gemittelte Verhältnis von effektiver Oberfläche des Brennerblocks und Anzahl dieser Bohrungen im Brennerblock für den Spülgasstrom in einem Bereich von 5 bis 50 cm² liegt.

3. Verfahren nach Anspruch 2, **dadurch gekennzeichnet, dass** der aus der Verteilervorrichtung austretende Spülgasstrom in Bezug auf die Mittelpunktsachse der Zuleitung radial verteilt wird.

4. Verfahren nach Ansprüchen 1 bis 3, **dadurch gekennzeichnet, dass** der Spülgasstrom über die gesamte effektive Oberfläche des Brennerblocks betrachtet in unterschiedlicher Menge zugeleitet wird, wobei in Nähe der den Feuerraum begrenzenden Wand eine höhere Menge zugeführt wird.

5. Verfahren nach Ansprüchen 1 bis 4, **dadurch gekennzeichnet, dass** man als Spülmedium Wasserdampf, Sauerstoff oder Gemische davon einsetzt.

6. Verfahren nach Ansprüchen 1 bis 5, **dadurch gekennzeichnet, dass** man zusätzlich die Innenwand des Feuerraums mit einem Spülgasstrom belegt.

7. Vorrichtung zur Durchführung eines der Verfahren gemäß Ansprüchen 1 bis 6, welche einen Reaktor enthaltend einen Brennerblock mit Feuerraum zur Acetylenherstellung umfasst, wobei der Brennerblock Bohrungen für die Zufuhr des Einsatzstoffgemisches und Bohrungen für die Zuleitung eines Spülgasstromes zur Spülung der feuerraumseitigen Oberfläche des Brennerblocks aufweist, wobei sich die effektive Oberfläche des Brennerblocks dadurch ergibt, dass man von der ursprünglichen feuerraumseitigen Oberfläche des Brennerblocks die durch die Bohrungen für Einsatzstoffgemisch entfallenden Teile dieser Fläche in Abzug bringt, wobei das gemittelte Verhältnis von effektiver Oberfläche des Brennerblocks und Anzahl dieser Bohrungen im Brennerblock für den Spülgasstrom in einem Bereich von 5 bis 100 cm² liegt und mittels einer am Ende der Bohrungen für den Spülgasstrom angrenzenden Verteilervorrichtung 70 - 100 Vol% des durch diese Bohrungen geleiteten Spülgasstromes parallel zur feuerraumseitigen Oberfläche des Brennerblocks geleitet wird.

## Claims

1. A process for preparing acetylene and synthesis gas by partial oxidation of hydrocarbons with oxygen, by first separately preheating the starting gases comprising a hydrocarbon-containing stream and an oxygen-containing stream and then mixing them in a mixing zone and, after they have flowed through the burner block, reacting them in the firing space and then cooling the products rapidly, wherein the effective surface area on the firing space side of the burner block which arises from the original surface area on the firing space side of the burner block, minus the parts of this area absent as a result of the bores for the feedstock mixture, is covered with a purge gas stream and this purge gas stream is introduced through the burner block by means of several bores, where the averaged ratio of effective surface area of the burner block to number of these bores in the burner block for the purge gas stream is within a range from 5 to 100 cm², where the averaged ratio of effective surface area of the burner block and number of these bores in the burner block for the purge gas stream is formed from the ratio of the total effective surface area of the burner block to the total number of bores for the purge medium, the purge gas stream conducted through the bores being deflected by means of distributor devices such that 70 to 100% by volume of the purge gas stream supplied is conducted parallel to the surface on the firing space side of the burner block.

2. The process according to claim 1, wherein the averaged ratio of effective surface area of the burner block to number of these bores in the burner block for the purge gas stream is within a range from 5 to 50 cm².

3. The process according to claim 2, wherein the purge gas stream exiting from the distributor device is distributed radially in relation to the center axis of the feed line.

4. The process according to claims 1 to 3, wherein the purge gas stream is supplied in different amounts viewed over the overall effective surface area of the burner block, a higher amount being supplied in the vicinity of the boundary wall of the firing space.

5. The process according to claims 1 to 4, wherein the purge medium used is steam, oxygen or mixtures thereof.

6. The process according to claims 1 to 5, wherein the inner wall of the firing space is additionally covered by a purge gas stream.

7. An apparatus for performing one of the processes according to claims 1 to 6, which comprises a reactor comprising a burner block with a firing space for acetylene production, the burner block having bores for the supply of the feedstock mixture and bores for the supply of a purge gas stream for purging of the surface on the firing space side of the burner block, the effective surface area of the burner block being calculated by subtracting the parts of this area absent as a result of the bores for the feedstock mixture from the original surface area on the firing space side of the burner block, the averaged ratio of effective surface area of the burner block to number of these bores in the burner block for the purge gas stream being within a range from 5 to 100 cm², and an adjoining distributor device for the purge gas stream at the end of the bores causing 70-100% by volume of the purge gas stream passed through these bores to be conducted parallel to the surface on the firing space side of the burner block.

## Revendications

1. Procédé pour la préparation d'acétylène et de gaz de synthèse par oxydation partielle d'hydrocarbures avec de l'oxygène, les gaz de départ, comprenant un flux hydrocarboné et un flux oxygéné, étant d'abord préchauffés séparément et ensuite mélangés dans une zone de mélange et amenés à réagir, après le passage du bloc brûleur, dans la chambre de combustion et ensuite refroidis rapidement, **caractérisé en ce qu'**on recouvre la surface effective côté chambre de combustion du bloc brûleur, qui est obtenue à partir de la surface initiale côté chambre de combustion du bloc brûleur, les parties supprimées par les trous pour le mélange de substances de départ étant déduites de cette surface, par un flux de gaz de rinçage, on introduit ce flux de gaz de rinçage au moyen de plusieurs trous dans le bloc brûleur, le rapport moyen de surface effective du bloc brûleur et du nombre de ces trous dans le bloc brûleur pour le flux de gaz de rinçage se situant dans une plage de 5 à 100 cm², le rapport moyen de surface effective du bloc brûleur et du nombre de ces trous dans le bloc brûleur pour le flux de gaz de rinçage étant formé à partir du rapport de la surface effective totale du bloc brûleur au nombre total de trous pour l'agent de rinçage, le flux de gaz de rinçage guidé au travers des trous étant dévié au moyen de dispositifs de distribution de manière telle que 70 à 100% en volume du flux de gaz de rinçage alimenté sont guidés parallèlement à la surface côté chambre de combustion du bloc brûleur.

2. Procédé selon la revendication 1, **caractérisé en ce que** le rapport moyen de la surface effective du bloc brûleur et du nombre de ces trous dans le bloc brûleur pour le flux de gaz de rinçage se situe dans une plage de 5 à 50 cm².

3. Procédé selon la revendication 2, **caractérisé en ce que** le flux de gaz de rinçage sortant du dispositif de distribution est distribué radialement par rapport à l'axe central de la conduite d'alimentation.

4. Procédé selon les revendications 1 à 3, **caractérisé en ce que** le flux de gaz de rinçage est alimenté en des quantités différentes par rapport à la surface effective totale du bloc brûleur, une quantité plus élevée étant alimentée à proximité de la paroi délimitant la chambre de combustion.

5. Procédé selon les revendications 1 à 4, **caractérisé en ce qu'**on utilise, comme agent de rinçage, de la vapeur d'eau, de l'oxygène ou des mélanges de ceux-ci.

6. Procédé selon la revendication 1 à 5, **caractérisé en ce qu'**on recouvre en outre la paroi interne de la chambre de combustion d'un flux de gaz de rinçage.

7. Dispositif pour la réalisation d'un des procédés selon les revendications 1 à 6, qui comprend un réacteur contenant un bloc brûleur présentant une chambre de combustion pour la préparation d'acétylène, le bloc brûleur présentant des trous pour l'alimentation en mélange de substances de départ et des trous pour l'introduction d'un flux de gaz de rinçage pour rincer la surface côté chambre de combustion du bloc brûleur, la surface effective du bloc brûleur étant obtenue en ce qu'on déduit, de la surface initiale côté chambre de combustion du bloc brûleur, les parties supprimées par les trous pour le mélange de substances de départ de cette surface, le rapport moyen de la surface effective du bloc brûleur et du nombre de ces trous dans le bloc brûleur pour le flux de gaz de rinçage étant situé dans une plage de 5 à 100 cm² et un dispositif distributeur adjacent à l'extrémité des trous pour le flux de gaz de rinçage guidant 70 à 100% en volume du flux de gaz de rinçage guidé au travers de ces trous parallèlement à la surface côté chambre de combustion du bloc brûleur.
